(19) Europäisches Patentamt
European Patent Office
Office européen des brevets

(11) **EP 2 481 401 A1**

(12) **EUROPEAN PATENT APPLICATION**

(43) Date of publication:
**01.08.2012 Bulletin 2012/31**

(21) Application number: **11176647.3**

(22) Date of filing: **14.01.2000**

(51) Int Cl.:
*A61K 9/50* (2006.01)      *A61K 38/00* (2006.01)
*A61K 39/00* (2006.01)      *A61K 9/10* (2006.01)
*A61K 9/113* (2006.01)      *A61K 9/16* (2006.01)
*A61K 38/21* (2006.01)      *A61K 39/39* (2006.01)
*A61K 38/27* (2006.01)

(84) Designated Contracting States:
**AT BE CH CY DE DK ES FI FR GB GR IE IT LI LU MC NL PT SE**

(30) Priority: **18.01.1999 KR 19990001232**
**21.12.1999 KR 19990059776**

(62) Document number(s) of the earlier application(s) in accordance with Art. 76 EPC:
**00900954.9 / 1 071 407**

(27) Previously filed application:
**14.01.2000 PCT/KR00/00025**

(71) Applicant: **LG Life Sciences, Ltd.**
**Seoul 150-721 (KR)**

(72) Inventors:
• **Kim, Myung, Jin**
**Yusong-gu,**
**Taejon 305-503 (KR)**

• **Kim, Sun, Jin**
**Kwangjin-gu,**
**Seoul 143-192**
**(KR)**
• **Kwon, Kyu, Chan**
**Yusong-gu,**
**Taejon 305-503 (KR)**
• **Kim, Joon**
**Yusung-gu,**
**Taejon 305-390 (KR)**

(74) Representative: **Krauss, Jan**
**Boehmert & Boehmert**
**Pettenkoferstrasse 20-22**
**80336 München (DE)**

<u>Remarks:</u>
This application was filed on 05-08-2011 as a divisional application to the application mentioned under INID code 62.

(54) **Lipophilic microparticles containing a protein drug or antigen and formulation comprising same**

(57)      A lipophilic microparticle having an average particle size ranging from 0.1 to 200 μm, comprising a lipophilic substance and an active ingredient selected from the group consisting of a protein or peptide drug and an antigen, retains the full activity of the active ingredient, and when formulated in the form of an oil dispersion or oil-in-water emulsion, it releases in an <u>in vivo</u> environment the active ingredient in a controlled manner over a long period.

EP 2 481 401 A1

**Description**

FIELD OF THE INVENTION

The present invention relates to microparticles coated with a lipophilic substance, which comprise a protein drug or an antigen, and a sustained-release formulation thereof for an effective in vivo delivery of the drug or antigen.

BACKGROUND OF THE INVENTION

It is well known that protein drugs or antigens suffer from the problem of denaturation caused by heat, organic solvents and/or unfavorable pH(Weiqi Lu et al., PDA J. Pharm. Sci. Tech., 49, 13-19 (1995)). They are usually administered by injection; however, because their in vivo activities last only for a short period of time after administration, they have to be administered repeatedly when a long-term treatment is required. For example, to treat a pituitary deficient child's dwarfism, human growth hormone(hGH) must be injected daily or every other day for a period of 6 months or more. Therefore, there has been many efforts to develop effective sustained-release formulations of protein drugs or antigens.

For example, extensive studies have been made to develop a sustained-release microparticle formulation prepared by coating a protein drug or an antigen with a synthetic biodegradable polymer, e.g., polylactide, polyglycolide, poly(lactide-co-glycolide), poly-ortho-ester or polyanhydride, which continuously releases the drug or the antigen as the polymer degrades in the body(M. Chasin and R. Langer, ed., Biodegradable Polymers as Drug Delivery Systems, Marcel Dekker (1990); and Heller, J., Adv. Drug Del. Rev., 10, 163 (1993)). Although this type of formulation has several advantages, it suffers from the serious problem that the drug or antigen undergoes denaturation upon its contact with an organic solvent during the preparation process thereof (Park, T. G. et al., J. Control. Rel., 33, 211-223 (1995)). The use of an organic solvent is unavoidable because a biodegradable polymer dissolves only in an organic solvent, e.g., methylene chloride, ethyl acetate, acetonitrile, chloroform or acetone.

In order to avoid such undesirable contact of a drug or antigen with an organic solvent, Lee et al. have prepared a microparticle by coating an antigen with a water-soluble polymer to obtain a primary particle; dispersing the primary particle in an organic solvent containing a biodegradable polymer; and drying the resulting dispersion to obtain a final microparticle(Lee, H. K. et al., J. Control. Rel., 44, 283-294 (1997); and US Patent No. 5,753,234). However, the process of preparing such microparticle is complicated and uneconomical.

There have also been attempts to develop a sustained release formulation containing a natural polymer, e.g., gelatin, collagen, chitosan, carboxymethyl cellulose, alginate or hyaluronic acid. The natural polymer easily absorbs water to form a gel having a high viscosity which releases a drug or antigen slowly. For example, US Patent No. 5,416,017 discloses a sustained-release injection formulation of erythropoietin containing a 0.01 to 3 % hyaluronic acid gel; Japanese Patent Laid-open No. 1-287041(1989), a sustained-release injection formulation of insulin containing a 1 % hyaluronic acid gel; Japanese Patent Laid-open No. 2-213(1990), a sustained-release formulation of calcitonin or human growth hormone containing a 5% hyaluronic acid gel; and Meyer, J. et al. (J. Controlled Rel., 35, 67 (1995)), a sustained release formulation of granulocyte-colony stimulating factor(G-CSF) containing a 0.5 to 4% hyaluronic acid gel.

Such hyaluronic acid gel formulations have a sustained release effect because the protein drugs slowly pass through the gel matrix having a high viscosity. However, a gel having a hyaluronic acid concentration of several % has a high viscosity, e.g., in the order of $10^5$ to $10^7$ centipoise, which makes the injection thereof difficult. Further, since both the drug and hyaluronic acid dissolve in water, a hyaluronic formulation is easily diluted by the body fluid after injection, with a consequential rapid release of the drug, usually within a day. For example, Japanese Patent Laid-open No. 1-287041(1989) discloses that when a 1 % hyaluronic acid gel formulation containing insulin was injected to a rabbit, the effect of lowering the blood glucose level was sustained for only 24 hours; and Meyer, J. et al. (vide supra) and US Patent No. 5,416,017, that when a 2 % hyaluronic acid gel formulation containing G-CSF and a 1.5 % hyaluronic acid gel formulation containing interferon-α together with serum protein are administered to an animal, blood levels of these protein drugs suddenly drop to below 1/10 of the initial levels in 24 hours.

Benzyl hyaluronate (HYAFF™, Fidia S.P.A.), a synthetic ester prepared by esterifying natural hyaluronic acid with benzyl alcohol, does not dissolve in water but in an organic solvent such as dimethyl sulfoxide(DMSO). A solid benzyl hyaluronate microparticle formulation containing a protein drug has been prepared by the emulsion-solvent extraction method(Nightlinger, N.S. et al., Proceed. Intern. Symp. Control Rel. Bioact. Mater., 22nd, Paper No., 3205 (1995); and Ilum, L. et al., J. Controlled Rel., 29, 133 (1994)), which is conducted by dissolving benzyl hyaluronate in DMSO; dispersing the protein drug in the resulting solution; adding the resulting dispersion to mineral oil to form an emulsion; and adding a solvent which is miscible with DMSO, e.g., ethyl acetate, to the emulsion to extract DMSO therefrom to obtain solid microparticles comprising the protein drug and benzyl hyaluronate.

However, the benzyl hyaluronate formulation has the drawback that the protein drug can be easily denatured by the organic solvent used in the preparative step and also by hydrophobic benzyl hyaluronate itself. An in vitro release test of a benzyl hyaluronate microparticle comprising granulocyte macrophage-colony stimulating factor(GM-CSF) showed that only 25 % of GM-CSF was released in the initial 2 days, and, thereafter no more of GM-CSF(Nightlinger, N.S. et al., vide supra), suggesting that most of the protein drug was denatured.

In the meantime, several attempts have been made to develop an oral formulation of a protein drug or an antigen, using, in particular, the fact that a microparticle containing an antigen having a particle size of 5 $\mu$m or less can be easily phagocytosized by a phagocyte. It, therefore, is expected that an antigen can be targeted to a specific location in the body, e.g., Payer's patches of small intestine.

However, it is difficult for M cells of Payer's patches to absorb an antigen due to its hydrophilicity and high molecular weight. To enhance the absorption of an antigen by M cells, an absorption enhancing agent such as a mixed bile salt-fatty acid micelle, chelator, fatty acid, phospholipid, acrylcarnitine, surfactant, medium chain glycerides has been employed (Lee, V. H. L., Crit . Rev. Ther. Drug Carrier Systems, 5, 69-97 (1988); Yoshioka, S. et al., J. Pharm. Sci., 71, 593-597 (1982); Scott-Moncrieff, J. C. et al., J. Pharm. Sci., 83, 1465-1469 (1994); Muranishi, S. et al., Chem. Pharm. Bull., 25, 1159-1163 (1977); Fix, J. A. et al., Am. J. Physiol., 251, G332-G340 (1986); Shao, Z. et al., Pharm. Res., 10, 143-250 (1993); Constantinides, P. P. et al., Proc. Int. Symp. Control. Release Bioactive Mater., 20, 184-185 (1993); and Bjork, E. et al., J. Drug Targeting, 2, 501-507 (1995)). Further, it has been reported that when a microparticle prepared by encapsulating bovine serum albumin in lecithin-cholesterol liposome is orally administered to an animal, an IgA response in the glandulae salivares is increased(Genco, R. et al., Ann. N. Y. Acad. Sci., 409, 650-667(1983)).

However, the only effective oral vaccine formulation that has been successfully developed is a polio vaccine for which an intestine receptor exists, and the liposome microparticle has the problem of structural instability.

SUMMARY OF THE INVENTION

Accordingly, it is an object of the present invention to provide a microparticle having an improved stability and effective delivery of a protein drug or an antigen.

It is another object of the present invention to provide a sustained-release formulation comprising said microparticle.

In accordance with one aspect of the present invention, there is provided a lipophilic microparticle having an average particle size ranging from 0.1 to 200 $\mu$m, comprising a lipophilic substance and an active ingredient selected from the group consisting of a protein or peptide drug and an antigen.

BRIEF DESCRIPTION OF THE DRAWINGS

The above objects and features of the present invention will become apparent from the following description of preferred embodiments taken in conjunction with the accompanying drawings, in which:

Fig. 1 shows in vitro release profiles of Microparticles 11, 12 and 13;
Figs. 2A and 2B reproduce RP HPLC scans of the Microparticle 12 extract solution obtained in Test Example 3 and a standard hGH aqueous solution, respectively; and
Figs. 3A and 3B illustrate SEC scans of the Microparticle 12 extract solution obtained in Test Example 3 and a standard hGH aqueous solution, respectively.

DETAILED DESCRIPTION OF THE INVENTION

The solid, lipophilic microparticle of the present invention comprises a lipophilic substance and an active ingredient.

The active ingredient which may be used in the present invention is a protein drug, peptide drug or antigen. Representative protein or peptide drugs include human growth hormone, bovine growth hormone, porcine growth hormone, growth hormone releasing hormone, growth hormone releasing peptide, granulocyte-colony stimulating factor, granulocyte macrophage-colony stimulating factor, macrophage-colony stimulating factor, erythropoietin, bone morphogenic protein, interferon, insulin, atriopeptin-III, monoclonal antibody, tumor necrosis factor, macrophage activating factor, interleukin, tumor degenerating factor, insulin-like growth factor, epidermal growth factor, tissue plasminogen activator and urokinase, but these do not limit the protein or peptide drugs which may be used in the present invention.

Representative antigens include those obtained from: one or more pathogens selected from the group consisting of adenovirus type 4&7, hepatitis A virus, hepatitis B virus, hepatitis C virus, influenza A & B virus, Japanese B encephalitis virus, measles virus, epidemic parotitis virus, rubella virus, polio virus, hydrophobia virus, chickenpox virus, yellow fever virus and human immunodeficiency virus; one or more pathogens selected from the group consisting of Bordetella pertussis, Borrelia burgdorferi, enterotoxigenic Escherichia coli, Haemophilus influenza type b, Mycobacterium leprae, Mycobacterium tuberculosis, Neisseria meningitidis A & C, Neisseria meningitidis B, Pseudomonas aeruginosa, Pseudomonas cepacia, Salmonella typhi, Shigella spp., Streptococcus pneumoniae and Vibrio cholerae; one or more pathogens selected from the group consisting of Coccidiodes immitis, Leishmania sp. and Plasmodium sp.; one or more pathogens responsible for the disease selected from the group consisting of bovine blackleg, bovine epidemic fever, bovine anthrax, bovine Akabane's disease, bovine foot-and-mouth disease, bovine mammitis, bovine infectious nasotracheal inflammation, bovine viral diarrhea, bovine infectious gastroenteritis, porcine cholera, porcine epidemic diarrhea, porcine atrophic gastritis, porcine disease caused by pavovirus, porcine enteritis caused by rotavirus, chicken Newcastle disease, chicken Marek's disease, chicken encephalomyelitis, rabies, dog distemper, dog enteritis caused by parvovirus and dog infectious hepatitis, the antigen being an attenuated, killed or recombinant antigen; or DNA, RNA, plasmid, CpG DNA or oligonucleotide extracted from the pathogen, but these do not limit the antigen which may be used in the present invention.

The lipophilic substance which may be used in the present invention includes a lipid and its derivatives, a fatty acid and its derivatives, a wax and a mixture thereof. Representative lipids include lecithin, phosphatidylcholine, phosphatidylehanolamine, phosphatidylserine and phosphatidylinositol. Representative derivatives of lipid include arachidoyl phosphatidylcholine and stearoyl phosphatidylcholine. Representative fatty acids include myristic acid, palmitic acid, stearic acid, and salts thereof. Representative derivatives of fatty acid include glyceryl stearate, sorbitan palmitate, sorbitan stearate, sorbitan monooleate and polysorbate. Representative waxes include an anionic emulsifying wax, carnauba wax and microcrystalline wax. Among those, preferred are surface-active lipophilic substances such as lecithin, phosphatidylcholine and phosphatidylehanolamine.

The lipophilic microparticle of the present invention may comprise 0.001 to 99 % by weight, preferably 0.1 to 10 % by weight, of an active ingredient and 1 to 99.999 % by weight, preferably 5 to 50 % by weight, of' an lipophilic substance, based on the weight of the microparticle.

In addition to the active ingredient, the microparticle of the present invention may further comprise hyaluronic acid or an inorganic salt thereof. Representative inorganic salts of hyaluronic acid include sodium hyaluronate, potassium hyaluronate, ammonium hyaluronate, calcium hyaluronate, magnesium hyaluronate, zinc hyaluronate and cobalt hyaluronate. Hyaluronic acid or its inorganic salt may be used in an amount ranging from 0.1 to 99 % by weight based on the weight of the microparticle.

The microparticle of the present invention may further comprise a water-soluble excipient for the purpose of stabilizing the active ingredient. The water-soluble excipient which may be used in the present invention includes a carbohydrate such as hydroxypropyl cellulose, carboxymethyl cellulose, sodium carboxymethyl cellulose, chitosan, alginate, glucose, xylose, galactose, fructose, maltose, saccharose, dextran and chondroitin sulfate; a protein such as albumin and gelatin; an amino acid such as glycine, alanine, glutamic acid, arginine, lysine and a salt thereof; a fatty acid such as stearic acid; an inorganic salt such as phosphate; a surfactant such as Tween® (ICI) poly(ethylene glycol) and a mixture thereof. The water-soluble excipient may be used in an amount ranging from 0.001 to 99 % by weight, preferably from 0.1 to 50 % by weight based on the amount of the microparticle.

The lipophilic microparticle of the present invention may be prepared by coating a solid particle containing an active ingredient with a lipophilic substance according to any one of following methods.

The lipophilic microparticle of the present invention may be prepared by dissolving an active ingredient in an aqueous solution containing other optional components such as hyaluronic acid and a water-soluble excipient; spray- or freeze-drying the resulting solution to obtain solid particles containing the active ingredient; dispersing the solid particles in an organic solvent containing a lipophilic substance; and then drying the dispersion to obtain solid, lipophilic microparticles. The organic solvent which may be used in the above procedure includes ethanol, methylene chloride and isopropyl alcohol.

Alternatively, the lipophilic microparticle of the present invention may be prepared by dissolving an active ingredient in an aqueous solution containing other optional components such as hyaluronic acid and a water-soluble excipient, adding a surface-active lipophilic substance such as lecithin thereto to allow the surface-active lipophilic substance to hydrate, and spray drying the resulting solution. In the spray-drying step, the surface-active lipophilic substance migrates to the surface of the droplets and coats the particles containing the active ingredient.

The inventive microparticle thus prepared has an average particle size ranging from 0.1 to 200 $\mu$m, preferably from 1 to 50 $\mu$m, more preferably 1 to 10 $\mu$m.

The inventive microparticle containing an active ingredient has several advantages: (1) the active ingredient is not denatured and retains its full activity; (2) it releases the active ingredient fully over a prolonged time; (3) it is easily dispersed in a lipophilic medium such as an oil, and the dispersion thus obtained has a low viscosity and retains the full activity of the active ingredient; (4) it is easily fused with the phospholipid membrane of the body to effectively transport the active ingredient into the body; (5) when it is orally administered, it can be translocated into M cells of Payers's patches located in the small intestine.

The microparticle of present invention may be formulated in the forms of dispersion, emulsion and aerosol.

Therefore, the present invention further provides a dispersion formulation prepared by dispersing the inventive lipophilic microparticles in a lipophilic medium. The lipophilic medium which may be used in the present invention includes an edible oil, mineral oil, squalene, squalane, cod liver oil, mono-, di- or triglyceride, and a mixture thereof. Representative edible oils include corn oil, olive oil, soybean oil, safflower oil, cotton seed oil, peanut oil, sesame oil, sunflower oil and a mixture thereof. The lipophilic medium may further comprise a dispersing agent or a preservative. The dispersion formulation may be used for injection or oral administration.

The present invention also provides an oil-in-water emulsion formulation comprising an aqueous injection medium and the dispersant formulation. The aqueous injection medium includes distilled water and a buffered solution. In the emulsion formulation, the lipophilic microparticles are coated with an oil and remain in the oil phase while enhancing the formation and stabilization of the water-in-oil emulsion. The emulsion formulation may be used for injection.

The emulsion formulation, when its active ingredient is an antigen, may further comprise another antigen in the aqueous injection medium, thereby providing a mixed vaccine formulation. For example, a dispersion formulation which comprises microparticles of hepatitis B surface antigen(HBsAg) dispersed in an edible oil, may be mixed with a DTP vaccine containing DTP antigen adsorbed to alum in an aqueous solution, to obtain an oil-in-water emulsion formulation. The emulsion formulation thus obtained is composed of an aqueous phase containing DTP antigen adsorbed to alum and an oil phase containing solid microparticles of HBsAg. In this emulsion formulation, HBsAg and DTP antigen are present separately in the aqueous phase and oil phase, respectively, thereby preventing undesirable interactions between HBsAg and DTP antigen. In contrast, a mixed vaccine formulation prepared by adsorbing several antigens to alum in an aqueous solution is known to suffer from a low vaccination effect due to undesirable interactions between the antigens.

The present invention further provides an aerosol formulation comprising the inventive microparticle. The aerosol formulation may be prepared according to a conventional method using a conventional excipient. The aerosol formulation may be administered via nasal or bronchial mucous membrane.

The following Examples are intended to further illustrate the present invention without limiting its scope.

Further, percentages given below for solid in solid mixture, liquid in liquid, and solid in liquid are on a wt/wt, vol/vol and wt/vol basis, respectively, unless specifically indicated otherwise.

Example 1: Preparation of Lipophilic Microparticles

Lecithin was added to 10 mM phosphate buffered solution(PBS) at a concentration of 2 %(w/v) and hydrated thoroughly. A recombinant hepatitis B surface antigen(HBsAg, LG chemical Ltd.) was added thereto to a concentration of 0.5 mg/mℓ and the resulting solution was provided to a spray dryer (Buchi 191) at a flow rate of 0.55 mℓ/minute to obtain solid microparticles(Microparticle 1). In this step, the inflow air temperature was 70 °C and the outflow air temperature, 50 °C. The particle size of the microparticles thus obtained was in the range of 0.1 to 5 $\mu$m.

Example 2: Preparation of Lipophilic Microparticles

Recombinant HBsAg was dissolved in 10 mM PBS to a concentration of 0.5 mg/mℓ and carboxymethylcellulose was added thereto to a concentration of 3 %(w/v). The resulting solution was provided to a spray dryer(Buchi 191) at a flow rate of 0.55 mℓ/minute to obtain primary particles. In this step, the inflow air temperature was 70 °C and the outflow air temperature, 50 °C.

A solution of 5 % (w/v) lecithin in ethanol was prepared and the primary particles were dispersed therein at a concentration of 5 %(w/v). The resulting dispersion was provided to a spray dryer(Buchi 191) at a flow rate of 1.0 mℓ/minute to obtain solid microparticles(Microparticle 2). In this step, the inflow air temperature was 85 °C and the outflow air temperature, 50 °C. The particle size of the microparticles thus obtained was in the range of 0.1 to 5 $\mu$m.

Example 3: Preparation of Lipophilic Microparticles

Carboxymethylcellulose was dissolved in 10 mM PBS to a concentration of 3 %(w/v). Lecithin was added thereto to a concentration of 2 %(w/v) and hydrated thoroughly. Recombinant HBsAg was then added thereto to a concentration of 0.5 mg/mℓ. The resulting solution was provided to a spray dryer(Buchi 191) at a flow rate of 0.55 mℓ/minute to obtain solid microparticles (Microparticle 3). In this step, the inflow air temperature was 70 °C and the outflow air temperature, 50 °C. The particle size of the microparticles thus obtained was in the range of 0.1 to 5 $\mu$m.

Examples 4 to 9: Preparation of Lipophilic Microparticles

The procedure of Example 3 was repeated using various ingredients listed in Table I to obtain various solid microparticles(Microparticles 4 to 9).

Table I

| Microparticle No. | Active Ingredient | Water-soluble Excipient | Lipophilic Substance | Particle Size ($\mu$m) |
|---|---|---|---|---|
| 1 | 0.5 mg/m$\ell$ HBsAg | - | 2 % (w/v) Lecithin | 0.1 - 5 |
| 2 | 0.5 mg/m$\ell$ HBsAg | 3 % (w/v) Carboxymethylcellulose | 5 % (w/v) Lecithin | 0.1 - 5 |
| 3 | 0.5 mg/m$\ell$ HBsAg | 3 % (w/v) Carboxymethylcellulose | 2 % (w/v) Lecithin | 0.1 - 5 |
| 4 | 0.5 mg/m$\ell$ HBsAg | 3 % (w/v) Carboxymethylcellulose | 1 % (w/v) Lecithin | 0.1 - 5 |
| 5 | 0.5 mg/m$\ell$ HBsAg | 3 % (w/v) Carboxymethylcellulose | 0.5 % (w/v) Lecithin | 0.1 - 5 |
| 6 | 0.5 mg/m$\ell$ HBsAg | 3 % (w/v) Hydroxypropylcellulose | 2 % (w/v) Lecithin | 0.1 - 5 |
| 7 | 0.5 mg/m$\ell$ HBsAg | 3 %(w/v) gelatin | 2 % (w/v) Lecithin | 0.1 - 5 |
| 8 | 0.5 mg/m$\ell$ HBpreS2Ag[1] | 3 % (w/v) Carboxymethylcellulose | 2 % (w/v) Lecithin | 0.1 - 5 |
| 9 | 2.16 mg/m$\ell$ SEC-SER[2] | 3 % (w/v) Carboxymethylcellulose | 2 % (w/v) Lecithin | 0.1 - 5 |

[1] Hepatitis B preS2 Antigen
[2] Staphylococcal Enterotoxin C1 mutant protein(LG Chemical Ltd.)(used in a quantity that the concentration of the protein becomes 6 wt% based on the total weight of ingredients)

Example 10: Preparation of Lipophilic Microparticles

Carboxymethylcellulose was dissolved in 10 mM PBS to a concentration of 3 %(w/v), the resulting solution was filtered and sterilized. Lecithin was added thereto to a concentration of 2 %(w/v) and hydrated thoroughly. A DNA extract of E. coli was dissolved in 10 mM PBS in an amount of 1 mg/m$\ell$ and this solution was added to the above mixture to a concentration of 6 wt% based on the total weight of the mixture, followed by mixing using a magnetic stirrer. The resulting suspension was provided to a spray dryer(Buchi 191) at a flow rate of 0.55 m$\ell$/minute to obtain solid microparticles(Microparticle 10). In this step, the inflow air temperature was 70 °C and the outflow air temperature, 50 °C. The particle size of the microparticles thus obtained was in the range of 0.2 to 3 $\mu$m.

Example 11: Preparation of Lipophilic Microparticles

Human growth hormone(hGH) was dissolved in 5 mM PBS to a concentration of 2 mg/m$\ell$ and then Tween 80 was added thereto in an amount of 0.01 wt% based on the weight of PBS. Sodium hyaluronate having a molecular weight of 1,000,000 was dissolved therein to a concentration of 0.2 %(w/v). The resulting solution was provided to a spray dryer(Buchi 190) at a flow rate of 3 m$\ell$/minute to obtain primary particles. In this step, the inflow air temperature was 85 °C. The average particle size of the primary particles thus obtained was 3 $\mu$m.
Lecithin was dissolved in ethanol to a concentration of 1 %(w/v) and then the primary particles were suspended therein at a concentration of 1 %(w/v). The resulting suspension was provided to a spray dryer(Buchi 190) to obtain microparticles(Microparticle 11). The average particle size of the microparticles thus obtained was 7 $\mu$m.

Examples 12 to 24: Preparation of Lipophilic Microparticles

The procedure of Example 11 was repeated using various ingredients listed in Table II to obtain various solid microparticles(Microparticles 12 to 24).

Table II

| Microparticle No. | Primary Particle | | | | | Microparticle | | | |
|---|---|---|---|---|---|---|---|---|---|
| | Active Ingredient | Water-soluble Excipient | Sodium Hyaluronate (%(w/v) / MW) | Inflow Air Temperature (°C) | Average Particle Size ($\mu$m) | Primary Particle (% (w/v)) | Lipophilic Substance | Solvent | Average Particle Size ($\mu$m) |
| 11 | 2 mg/m$\ell$ hGH | 0.01 wt% Tween 80 | 0.2 %(w/v) / MW: 1,000,000 | 85 | 3 | 1 | 1 %(w/v) Lecithin | Ethanol | 7 |
| 12 | 1 mg/m$\ell$ hGH | 0.01 wt% Tween 80 | 0.1 %(w/v) / MW: 2,000,000 | 85 | 2 | 1 | 1 %(w/v) Lecithin | EthanoL | 5 |
| 13 | 0.1 mg/m$\ell$ hGH | 0.01 wt% Tween 80 | 0.09 %(w/v) / MW: 2,000,000 | 85 | 2 | 1 | 1 %(w/v) Lecithin | EthanoL | 5 |
| 14 | 2 mg/m$\ell$ bST[a] | 0.01 wt% Tween 80 | 0.2 %(w/v) / MW: 2,000,000 | 85 | 3 | 2 | 1 %(w/v) Lecithin | Ethanol | 7 |
| 15 | 2 mg/m$\ell$ pST[b] | 0.01 wt% Tween 80 | 0.2 %(w/v) / MW: 2,000,000 | 85 | 3 | 1 | 1 %(w/v) Lecithin | Ethanol | 7 |
| 16 | 0.4 mg/m$\ell$ GM-CSF[C] | 0.01 wt% Tween 80 | 0.16 %(w/v) / MW: 2,000,000 | 85 | 3 | 1 | 1 %(w/v) Lecithin | Ethanol | 7 |
| 17 | 1,000 IU/m$\ell$ EPO[d] | 0.01 wt% Tween 80, & 0.5 mg/m$\ell$ Serum Albumin | 0.25 %(w/v) / MW: 2,000,000 | 85 | 3.5 | 1 | 1 %(w/v) Lecithin | Ethanol | 7 |

| Microparticle No. | Primary Particle | | | | | Microparticle | | | |
|---|---|---|---|---|---|---|---|---|---|
| | Active Ingredient | Water-soluble Excipient | Sodium Hyaluronate (%(w/v) / MW) | Inflow Air Temperature (°C) | Average Particle Size (μm) | Primary Particle (% (w/v)) | Lipophilic Substance | Solvent | Average Particle Size (μm) |
| 18 | 200,000IU/mℓ Interferon-α | 0.01 wt% Tween 80, 0.2 mg/mℓ D-mannitol, & 0.2 mg/mℓ Serum Albumin | 0.25 %(w/v) / MW: 2,000,000 | 105 | 3.5 | 1 | 1 %(w/v) Lecithin | Ethanol | 7 |
| 19 | 200,000IU/mℓ Interferon-Γ | 0.01 wt% Tween 80, 0.2 mg/mℓ Glycine,&0.2 mg/mℓ Serum Albumin | 0.25 %(w/v) / MW: 2,000,000 | 105 | 3.5 | 1 | 1 %(w/v) Lecithin | EthanoL | 7 |
| 20 | 20 IU/mℓ Insulin | 0.01 wt% Tween 80 | 0.2 %(w/v) / MW: 2,000,000 | 85 | 3 | 1 | 1 %(w/v) Lecithin | Ethanol | 7 |
| 21 | 2 mg/mℓ IGF-I[e)] | 0.01 wt% Tween 80 | 0.2 %(w/v) / MW: 2,000,000 | 85 | 3 | 1 | 1 %(w/v) Lecithin | Ethanol | 7 |
| 22 | 1 mg/mℓ hGH | 0.01 wt% Tween 80 | 0.1 %(w/v) / MW: 2,000,000 | 85 | 2 | 1 | 0.5 %(w/v) Stearic Acid | Methylene Chloride | 6 |
| 23 | 1 mg/mℓ hGH | 0.01 wt% Tween 80 | 0.1 %(w/v) / MW: 2,000,000 | 85 | 2 | 1 | 0.5 %(w/v) sorbitan Monostearate | Isopropyl Alcohol | 7 |

(continued)

| Microparticle No. | Primary Particle | | | | | Microparticle | | | |
|---|---|---|---|---|---|---|---|---|---|
| | Active Ingredient | Water-soluble Excipient | Sodium Hyaluronate (%(w/v) / MW) | Inflow Air Temperature (°C) | Average Particle Size ($\mu$m) | Primary Particle (% (w/v)) | Lipophilic Substance | Solvent | Average Particle Size ($\mu$m) |
| 24 | 1 mg/m$\ell$ hGH | 0.01 wt% Tween 80 | 0.1 %(w/v) / MW: 2,000,000 | 85 | 2 | 1 | 0.5 %(w/v) Glyceryl Monosearate | Methylene Chloride | 7 |

[a] bovine somatotropin

[b] porcine somatotropin

[c] granulocyte macrophage-cotony stimulating factor

[d] erythropoietin

[e] insulin-like growth factor-I

Example 25: Preparation of Cotton Seed Oil Dispersion Formulations of Microparticle 3

Microparticle 3 prepared in Example 3 was added to cotton seed oil and dispersed using a magnetic stirrer to obtain five cotton seed oil dispersions containing 20, 50, 100, 200 an 500 mg/mℓ of Microparticle 3, respectively.

Example 26: Preparation of Edible Oil Dispersion Formulations of Microparticle 3

The procedure of Example 25 was repeated using soybean oil, corn oil and sesame oil, respectively, to obtain a soybean oil, corn oil and sesame oil dispersions each containing 100 mg/mℓ of Microparticle 3.

Example 27: Preparation of Edible Oil Dispersion Formulations of Microparticle 8

The procedure of Example 25 was repeated using Microparticle 8 to obtain cotton seed oil, soybean oil, corn oil and sesame oil dispersions each containing 100 mg/mℓ of Microparticle 8.

Example 28: Preparation of Edible Oil Dispersion Formulations of Microparticle 9

The procedure of Example 25 was repeated using Microparticle 9 to obtain cotton seed oil, soybean oil, corn oil and sesame oil dispersions each containing 100 mg/mℓ of Microparticle 9.

Example 29: Preparation of Edible Oil Dispersion Formulations of Microparticle 10

The procedure of Example 25 was repeated using Microparticle 10 to obtain cotton seed oil, soybean oil, corn oil and sesame oil dispersions each containing 100 mg/mℓ of Microparticle 10.

Example 30: Preparation of Cotton Seed Oil Dispersion Formulations of Microparticle 12

The procedure of Example 25 was repeated using Microparticle 12 to obtain five cotton seed oil dispersions each containing 50, 100, 200, 360 and 500 mg/mℓ of Microparticle 12, respectively.

Example 31: Preparation of Edible Oil Dispersion Formulations of Microparticle 12

The procedure of Example 25 was repeated using Microparticle 12 to obtain soybean oil, corn oil and sesame oil dispersions each containing 100 mg/mℓ of Microparticle 12.

Example 32: Preparation of Edible Oil Dispersion Formulations of Microparticle 14

The procedure of Example 25 was repeated using Microparticle 14 to obtain cotton seed oil, soybean oil, corn oil and sesame oil dispersions each containing 360 mg/mℓ of Microparticle 14.

Example 33: Preparation of Edible Oil Dispersion Formulations of Microparticle 15

The procedure of Example 25 was repeated using Microparticle 15 to obtain cotton seed oil, soybean oil, corn oil and sesame oil dispersions each containing 360 mg/mℓ of Microparticle 15.

Example 34: Preparation of Edible Oil Dispersion Formulations of Microparticle 18

The procedure of Example 25 was repeated using Microparticle 18 to obtain cotton seed oil, soybean oil, corn oil and sesame oil dispersions each containing 360 mg/mℓ of Microparticle 18.

Example 35: Preparation of Emulsion Formulations of Microparticle 3

Each of the soybean oil, corn oil and sesame oil dispersions obtained in Example 26 was added to a 4-fold volume of 0.9 % NaCl solution to obtain a mixture of oil and water(1:4) containing 20 mg/mℓ Microparticle 3. The resulting mixture was mixed to obtain a homogeneous, white-opaque oil-in-water emulsion.

Example 36: Preparation of Emulsion Formulations of Microparticle 12

The procedure of Example 35 was repeated using the cotton seed dispersions obtained in Example 30 to obtain five homogeneous, white-opaque oil-in-water emulsions, which respectively contained 5, 20, 50, 120 and 200 mg/m$\ell$ of Microparticle 12, the oil to the water ratios thereof being 1:9, 1:4, 1:3, 1:2, and 2:3, respectively.

In these emulsion formulations, the solid microparticles were dispersed in the oil phase and the oil droplets containing the microparticles were stable due to the lipophilic surface of the microparticles. The emulsions were stable at an ambient temperature for a period of over 2 weeks.

Example 37: Preparation of Emulsion Formulations of Microparticle 12

The procedure of Example 35 was repeated using the soybean oil, corn oil and sesame oil dispersions obtained in Example 31 to obtain homogeneous, white-opaque oil-in-water emulsion formulations.

Example 38: Preparation of Emulsion Formulation of Microparticle 22

The procedure of Example 25 was repeated using Microparticle 22 and soybean oil to obtain a soybean oil dispersion containing 100 mg/m$\ell$ of Microparticle 22. Using the resulting dispersion, the procedure of Example 35 was repeated to obtain a homogeneous, white-opaque oil-in-water emulsion formulation.

Example 39: Preparation of Emulsion Formulation of Microparticle 23

The procedure of Example 25 was repeated using Microparticle 23 and soybean oil to obtain a soybean oil dispersion containing 100 mg/m$\ell$ of Microparticle 23. Using the resulting dispersion, the procedure of Example 35 was repeated to obtain a homogeneous, white-opaque oil-in-water emulsion formulation.

Example 40: Preparation of Emulsion Formulation of Microparticle 24

The procedure of Example 25 was repeated using Microparticle 24 and soybean oil to obtain a soybean oil dispersion containing 100 mg/m$\ell$ of Microparticle 24. Using the resulting dispersion, the procedure of Example 35 was repeated to obtain a homogeneous, white-opaque oil-in-water emulsion formulation.

Example 41: Preparation of Emulsion Formulations of Microparticle 14

The procedure of Example 35 was repeated using the cotton seed oil, soybean oil, corn seed oil and sesame oil dispersions obtained in Example 32 and 2-fold volumes of 0.9 % NaCl solution to obtain four homogeneous, white-opaque oil-in-water emulsion formulations. Each of the formulations thus obtained contained 120 mg/m$\ell$ of Microparticle 14 in a mixture of oil and water(1:2).

Example 42: Preparation of Emulsion Formulations of Microparticle 15

The procedure of Example 35 was repeated using the cotton seed oil, soybean oil, corn seed oil and sesame oil dispersions obtained in Example 33 and 2-fold volumes of 0.9 % NaCl solution to obtain four homogeneous, white-opaque oil-in-water emulsion formulations. Each of the formulations thus obtained contained 120 mg/m$\ell$ of Microparticle 15 in a mixture of oil and water(1:2).

Example 43: Preparation of Emulsion Formulations of Microparticle 18

The procedure of Example 35 was repeated using the cotton seed oil, soybean oil, corn seed oil and sesame oil dispersions obtained in Example 34 and 2-fold volumes of 0.9 % NaCl solution to obtain homogeneous, white-opaque oil-in-water emulsion formulations of Microparticle 18. Each of the formulations thus obtained contained 120 mg/m$\ell$ of Microparticle 18 in a mixture of oil and water(1:2).

Example 44: Preparation of Emulsion Formulations of Microparticle 3

The procedure of Example 35 was repeated except that alum was used in place of 0.9 % NaCl, to obtain homogeneous, white-opaque oil-in-water emulsion formulations.

Comparative Example 1: Preparation of Comparative Microparticle 1

The procedure for Example 2 was repeated without the use of lecithin to obtain comparative microparticles coated with no lipophilic substance(Comparative Microparticle 1)

Comparative Example 2: Preparation of Dispersion Formulation of Comparative Microparticle 1

The procedure of Example 25 was repeated using Comparative Microparticle 1 and soybean oil to obtain a soybean oil dispersion containing 100 mg/mℓ of Comparative Microparticle 1 (Comparative Dispersion 1), which contained aggregated, non-homogeneously dispersed microparticles.

Comparative Example 3: Preparation of Emulsion Formulation of Comparative Microparticle 1

The procedure of Example 35 was repeated using Comparative Dispersion 1 and physiological saline for injection to obtain an emulsion formulation(Comparative Emulsion 1), which was not a homogeneous emulsion but showed a separation with aggregated microparticles.

Comparative Example 4: Preparation of Comparative Microparticle 2

The procedure of Example 11 was repeated using 1 mg/mℓ hGH, 0.1 %(w.v) sodium hyaluronate having a molecular weight of 2,000,000 to obtain comparative microparticles having no lipophilic coating(Comparative Microparticle 2).

Comparative Example 5: Preparation of Dispersion Formulation of Comparative Microparticle 2

The procedure of Example 25 was repeated using Comparative Microparticle 2 and cotton seed oil to obtain a cotton seed oil dispersion containing 100 mg/mℓ of Comparative Microparticle 2(Comparative Dispersion 2).

Comparative Example 6: Preparation of Emulsion Formulation of Comparative Microparticle 2

The procedure of Example 35 was repeated using Comparative Dispersion 2. The resulting mixture (Comparative Emulsion 2) did not form a homogeneous emulsion and but exhibited a phase separation, with the microparticles dispersed in both the oil and water phases.

Comparative Example 7: Preparation of Comparative Microparticle 3

The procedure of Example 11 was repeated using $2 \times 10^5$ IU/mℓ interferon-$\alpha$, 0.2 mg/mℓ D-mannitol, 0.2 mg/mℓ serum albumin and 0.25 %(w/v) sodium hyaluronate having a molecular weight of 2,000,000 at an inflow air temperature of 105 °C to obtain a comparative microparticles having no lipophilic coating (Comparative Microparticle 3) which had an average particle size of 3.5 $\mu$m.

Comparative Example 8: Preparation of Oil Dispersion Formulation of Comparative Microparticle 3

The procedure of Example 25 was repeated using Comparative Microparticle 3 and cotton seed oil to obtain a cotton seed oil dispersion containing 100 mg/mℓ of Comparative Microparticle 3(Comparative Dispersion 3).

Comparative Example 9: Preparation of Emulsion Formulation of Comparative Microparticle 3

The procedure of Example 35 was repeated using Comparative Dispersion 3. The resulting mixture (Comparative Emulsion 3) did not form a homogeneous emulsion but exhibited a phase separation, with the microparticles dispersed in both the oil and water phases.

Test Example 1: Stability Test of Microparticles 1 and 3

To examine whether an antigen contained in a microparticle maintains its activity, each of Microparticles 1 and 3 was dissolved in water and diluted with 1,000- to 100,000-fold volume of water and the HBsAb antigenicity was examined using AUZYME kit(Abbott, USA). Results are shown in Table III.

Table III

| Microparticle No. | Antigenicity Before Preparation (%) | Antigenicity After Preparation (%) |
|---|---|---|
| 1 | 89.5 | 86.7 |
| 3 | 88.6 | 82.3 |

As can be seen from Table III, the antigenicity of HBsAg contained in the inventive microparticles was largely unchanged before and after the preparation. Therefore, the antigen contained in the microparticle of the present invention preserves its antigenicity.

Test Example 2: In Vitro Release Test of Microparticles 11, 12 and 13

Each of Microparticles 11, 12 and 13 was dispersed in a buffered solution(150 mM NaCl, 10 mM phosphate, 0.05 % sodium azide, pH 7.4) so that the hGH concentration was 1.0 mg/mℓ. An in vitro release test was conducted by stirring the resulting dispersion at 37 °C and at a preset time, the dispersion was centrifuged at 800 g for 10 minute and a sample of the supernatant, taken in an amount of 1/10 volume of the dispersion, was mixed with an equal volume of the above buffered solution. hGH contained in the supernatant was quantified using Lowry method with High Performance Liquid Chromatography(HPLC).

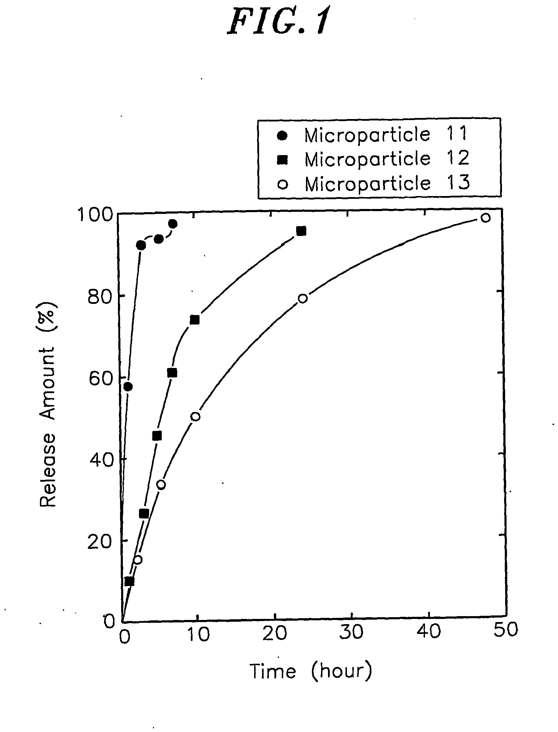

Fig. 1 shows the release profiles of Microparticles 11, 12 and 13 thus determined. As can be seen from Fig. 1, as the molecular weight of hyaluronic acid becomes higher, and as the content of hGH decrease, the release rate of hGH becomes slower. Therefore, the protein drug release rate can be controlled by adjusting the molecular weight of hyaluronic acid and the protein drug content. Further, in vitro release test results showed that an initial burst release of the protein drug does not occur and the release rate is constant until 70 % of protein is released.

Test Example 3: Stability test of Microparticle 12

To examine whether hGH contained in Microparticle 12 maintains its activity, the procedure of Test Example 1 was repeated and the amount of hGH released from Microparticle 12 in 48 hours was determined with both reversed phase(RP) HPLC and size exclusion chromatography(SEC). The RP HPLC was used to assess the extent of oxidative and deamidative denaturation of the protein; and SEC, the denaturation of the protein by aggregation.

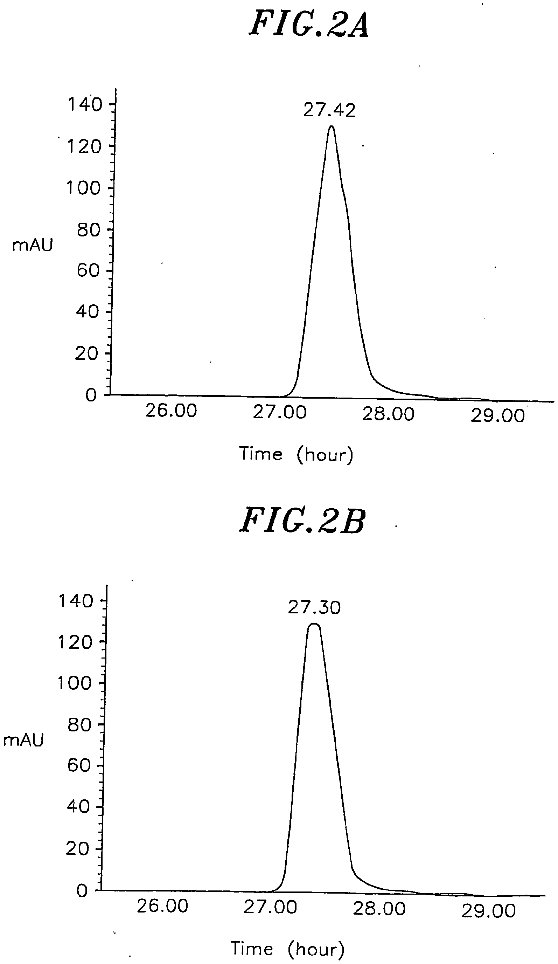

Figs. 2A and 2B represent RP HPLC results for the Microparticle 12 extract solution and the hGH aqueous solution used in preparation of Microparticle 12, respectively.

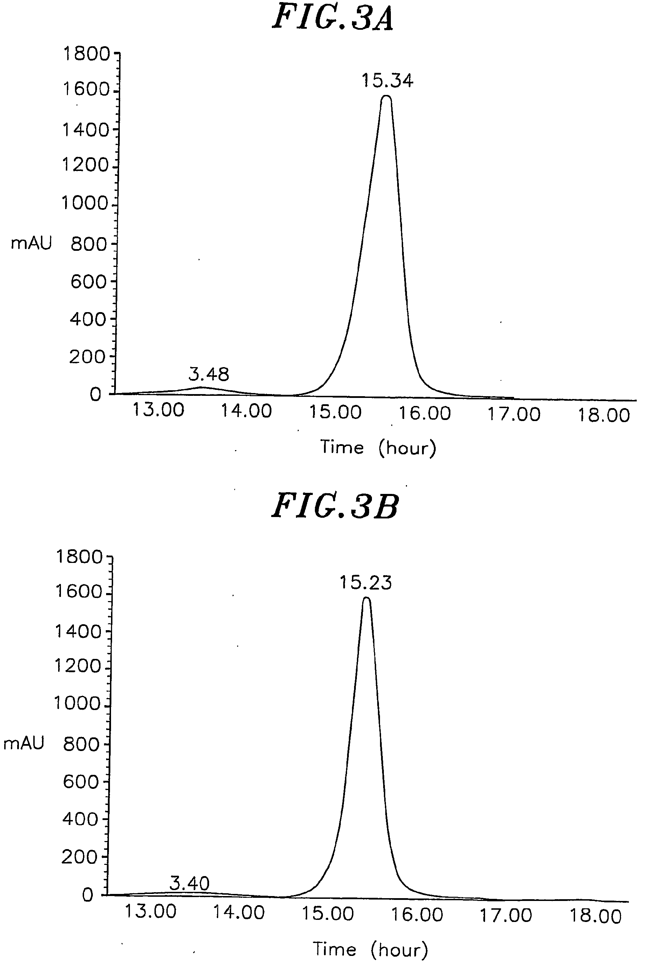

Figs. 3A and 3B provide SEC results for the Microparticle 12 extract solution and hGH aqueous solution used in the preparation of Microparticle 12, respectively.

As can be seen from Figs. 2 and 3, the amount of hGH released from the inventive microparticle is identical with that of the original hGH aqueous solution according to the RP HPLC results; and the SEC results show the monomeric hGH content is 95 % or more. These results suggest that hGH is not denatured during the preparation of the inventive microparticles.

Test Example 4: In Vitro Release Test of Microparticles 14 to 21

The procedure of Test Example 2 was repeated using each of Microparticles 14 to 21, and the cumulative amounts of the drug released in 10 and 72 hours as well as the monomeric protein content of the 72 hours sample were determined. The results are shown in Table IV.

Table IV

| Microparticle No. | Released Amount (%) at 10 hours | Released Amount (%) at 72 hours | Monomer Content (%) |
|---|---|---|---|
| 14 | 45 | 92 | 94 |
| 15 | 47 | 87 | 89 |
| 16 | 44 | 89 | 97 |
| 17 | 32 | 76 | 95 |
| 18 | 54 | 92 | 94 |

(continued)

| Microparticle No. | Released Amount (%) at 10 hours | Released Amount (%) at 72 hours | Monomer Content (%) |
|---|---|---|---|
| 19 | 49 | 88 | 95 |
| 20 | 60 | 95 | 95 |
| 21 | 38 | 93 | 97 |

As can be seen from Table IV, the microparticles of the present invention release the protein drug over a period of 3 days, with no sign of denaturation of the protein drug.

Test Example 5: Injectability Test 1

To examine whether the microparticles of the present invention are homogeneously dispersed in an oil dispersion or in an oil-water emulsion, injectability tests were conducted. The injectability is defined as the force required to push a syringe filled with a test sample at a velocity of 80 mm/minute. A 23 gauge injection needle was used. Samples used in these tests were an alum dispersion prepared by diluting alum with 20-fold PBS; soybean oil; the soybean oil dispersion of Example 26; the emulsion of Example 44, Comparative Dispersion 1 and Comparative Emulsion 1, and the results are shown in Table V.

Table V

| Formulation | Conc. of Microparticle (mg/m$\ell$) | Injectability (kg$_f$) |
|---|---|---|
| Alum Dispersion | 0 | 0.1 |
| Soybean Oil | 0 | 0.5 |
| Dispersion Of Example 26 | 50 | 0.1 |
| Emulsion Of Example 44 | 20 | 0.5 |
| Comparative Dispersion 1 | 50 | Not Injected |
| Comparative Emulsion 1 | 20 | Not Injected |

As can be seen from Table V, the dispersion of Example 26 is easily injectable, showing that Microparticle 3 of the present invention are well dispersed in oil as compared with Comparative Microparticle 1 having no lecithin coating. Particularly, since the emulsion of the present invention has a superior injectability, it can be used in preparing a mixed formulation.

Test Example 6: Injectability Test 2

The procedure of Test Example 5 was repeated for 0.9 % NaCl aqueous solution, cotton seed oil, the cotton seed oil dispersion of Example 30, the emulsion of Example 36, Comparative Dispersion 2, and Comparative Emulsion 2, using a 26 gauge syringe needle. Results are shown in Table VI.

Table VI

| Formulation | Conc. of Microparticle (mg/m$\ell$) | Injectability (kg$_f$) |
|---|---|---|
| 0.9 % NaCl Aqueous Solution | 0 | 0.1 |
| Cotton seed Oil | 0 | 1.6 |
| Dispersion Of Example 30 | 100 | 1.7 |
| Emulsion Of Example 36 | 120 | 0.8 |
| Comparative Dispersion 2 | 100 | 10.5 |
| Comparative Emulsion 2 | 20 | 23.3 |

As can be seen from Table VI, Microparticle 12 of the present invention has good dispersibility in cotton seed

EP 2 481 401 A1

oil due to the lipophilic lecithin coating, and the cotton seed oil dispersion of the present invention has an injectability equivalent to that of the cotton seed oil. Further, the emulsion of Example 36 has a lower injectability than cotton seed oil in spite of the high concentration of Microparticle 12.

In contrast, Comparative Microparticle 2 has an inferior dispersibility in cotton seed oil due to the absence of a lipophilic coating, causing the poor injectability of Comparative Dispersion 2. Further, when the hydrophilic surface of Comparative Microparticle 2 caused a phase separation in Comparative Emulsion 2, with a consequential leaching of the sodium hyaluronate component into the aqueous phase to raise the viscosity of water layer. Therefore, Comparative Emulsion 2 has an even poorer injectability than Comparative Dispersion 2.

Test Example 7: Injectability Test 3

The procedure of the Test Example 5 was repeated for the emulsions obtained in Examples 38 to 40, and soybean oil as a control. The injectability of soybean oil was 1.4 $kg_f$, and those of the emulsions obtained in Examples 38 to 40 were in the range of 0.3 to 0.5 $kg_f$ similar to that of 0.9 % NaCl aqueous solution. The lipophilic surface of the microparticles of the present invention becomes coated with soybean oil, resulting in a homogeneous oil-in-water emulsion which has an injectability equivalent to that of water.

Test Example 8: Injection of Formulation to Animal

To examine the biological activity of the formulation of the present invention on injection, Microparticle 3 was added to soybean oil to prepare four dispersions having recombinant HBsAg loadings of 0.5, 0.125, 0.03125 and 0.0078 $\mu$g protein/m$\ell$, respectively. As a comparative formulation, a hepatitis B vaccine(LG Chemical Ltd., Korea) containing alum as an immune adjuvant was diluted with PBS to prepare samples having HBsAg loadings of 0.5, 0.125, 0.03125, and 0.0078 $\mu$g protein/m$\ell$, respectively.

Each dispersion sample was injected intraperitoneally to 4 weeks-old male Balb/C (H-2d) mice(10 mice) and blood samples were taken therefrom 4 months after the injection. Serums were obtained from the blood samples and titers of antibody thereof and antibody formation(%) were determined using AUSAB EIA kit(Abbott, USA) and $ED_{50}$($\mu$g) of the microparticle was calculated using a statistical method(probit analysis). Results are shown in Table VII.

Table VII

| Dose(mIU/m$\ell$) Formulation | 0.5 | 0.125 | 0.0312 | 0.0078 | $ED_{50}$($\mu$g) |
|---|---|---|---|---|---|
| Microparticle 3 | 106.35 | 38.98 | 13.32 | 4.68 | 0.0121 |
| Comparative Formulation | 12.43 | 6.1 | 4.32 | 3.15 | 0.1504 |

As can be seen from Table VII, the microparticle of the present invention has a smaller amount of $ED_{50}$ than the comparative formulation and higher titer of antibody than comparative formulation when the same amount of antigen is injected. Therefore, the microparticle of the present invention functions as an adjuvant for superior antibody formation.

Test Example 9: Oral Administration of Formulation to Animal

To examine the biological activity of the formulation of the present invention on oral administration, Microparticle 3 was added to soybean oil to prepare a dispersion having a recombinant HBsAg loading of 5 $\mu$g protein/m$\ell$. As a comparative formulation, a hepatitis B vaccine(LG Chemical Ltd., Korea) was diluted with PBS to obtain the same loading of 5 $\mu$g protein/m$\ell$.

Each dispersion sample was orally administered to 4 weeks-old male Balb/C (H-2d) mice (10 mice), and administered two more times, 2 and 4 weeks thereafter. Blood samples were taken from the mice after 8 weeks. Serums was obtained from the blood samples and titers of antibody and antibody formation(%) were determined using AUSAB EIA kit(Abbott, USA). Results are shown in Table VIII

Table VIII

| Formulation | Antibody Formation(%) | Titer of Antibody (mIU/m$\ell$) |
|---|---|---|
| Microparticle 3 | 80 | 10.1 |
| Comparative Formulation | 0 | 0 |

As can be seen from Table VIII, the mice administered with the microparticle of the present invention had 80 % antibody formation while the mice administered with the comparative formulation did not form antibody. Therefore, the microparticle of the present invention has a superior ability for forming antibody in vivo even when it is orally administered. This suggests that the microparticle of the present invention can be advantageously employed in a formulation for oral administration.

Test Example 10: Cytotoxic Lymphocyte Test

To examine whether the microparticle of the present invention induces a cell-mediated immune response, Microparticle 3 was added to soybean oil to obtain a dispersion having a loading of 10 $\mu$g protein/m$\ell$. As a comparative formulation, a hepatitis B vaccine(LG Chemical Ltd., Korea) was diluted with PBS to the same loading of 10 $\mu$g protein/m$\ell$.

Each dispersion formulation was subcutaneously administered to 4 weeks-old male Balb/C (H-2d) mice (10 mice), and administered two more times thereafter at 2 months intervals. Two weeks after the last administration, pre cytotoxic lymphocyte(CTL) cells were obtained from the immunized mice and cultured to obtain effector(E) cells. E cells were cultured together with target(T) cells varying the E cell to T cell ratio, and then, the number of T cells lysed by E cells(specific lysis(%)) was determined as follow.

$$\text{Specific Lysis(\%)} = \frac{\text{cpm}_{\text{sample}} - \text{cpm}_{\text{natural release}}}{\text{cpm}_{\text{maximum release}} - \text{cpm}_{\text{natural release}}} \times 100$$

A high specific lysis value means that the cell-mediated immune response is actively induced. Results are shown in Table IX.

Table IX

| E:T<br>Formulation | 20:1 | 5:1 | 1:1 |
|---|---|---|---|
| Microparticle 3 | 20 % | 8 % | 2 % |
| Comparative Formulation | 2 % | 1 % | 2 % |

As can be seen from Table IX, the microparticle of the present invention induced the cell-mediated immune response while the comparative formulation did not.

Test Example 11: Animal Test

To examine the immune enhancing effect of the microparticle of the present invention, Microparticle 9 was added to soybean oil to obtain a dispersion having a SEC-SER loading of 4.0 mg protein/m$\ell$. As a comparative formulation, SEC-SER antigen was mixed with a commercial adjuvant ISA according to the manufacturer's guide, and then, diluted to the same loading of 4.0 mg protein/m$\ell$. As a control, microparticles prepared by repeating the procedure of Example 9 except that the antigen was omitted, was dispersed in soybean oil.

Each dispersion formulation was intramuscularly injected to 2nd or 3rd lactating period cows(5 cows) having a large number of somatic cells and injected two more times, 2 and 4 weeks thereafter. Blood samples were taken from the cows after 2, 6, 10 and 14 weeks and then the titer of antibody was determined. Results expressed in ELISA reader are shown in Table X.

Table X

| | Before Injection | 2 weeks | 6 weeks | 10. weeks | 14 weeks |
|---|---|---|---|---|---|
| Comparative Formulation | 1 | 2.552 | 2.667 | 2.466 | 2.322 |
| Microparticle 9 | 1 | 4.472 | 5.123 | 5.911 | 4.522 |
| Control | 1 | 1.335 | 1.486 | 2.002 | 2.278 |

As can be seen from Table X, the formulation of the present invention induced the formation of a high level of antibody which lasted for 14 weeks after the first injection. Therefore, the formulation of the present invention can be advantageously employed as an animal vaccine.

Test Example 12: Animal Test

The effect administering of the inventive formulation containing hGH was examined using 7 weeks-old female dwarf rats (body weight: approximate 100g) which have the heredity of low growth hormone secretion.

The dispersion of Examples 30, the emulsion of Example 36, Comparative Dispersion 2, Comparative Emulsion 2, and Utroprin[R](LG Chemical Ltd., Korea), an aqueous formulation were chosen for the test, and each was administered to a group of 10 dwarf rats at an hGH dose of 350 $\mu$g per rat and then the weight gain was examined. As a control, rats which did not receive hGH were used. The average cumulative net weight gains are shown in Table XI.

Table XI

| | | | | | | (unit: g) |
|---|---|---|---|---|---|---|
| Day | 1 | 2 | 3 | 4 | 5 | 6 |
| Control | 0.9 | 2.7 | 3.6 | 4.7 | 6.3 | 7.5 |
| Utropin® | 4.7 | 4.2 | 5.3 | 6.4 | 7.1 | 8.5 |
| Comparative Dispersion 2 | 5.0 | 5.7 | 7.2 | 8.5 | 10.2 | 11.5 |
| Comparative Emulsion 2 | 4.3 | 4.9 | 3.6 | 5.4 | 6.7 | 7.8 |
| Dispersion of Example 30 | 5.5 | 6.6 | 7.3 | 8.7 | 11.4 | 12.3 |
| Emulsion of Example 36 | 5.3 | 6.8 | 8.1 | 9.2 | 11.3 | 13.0 |

As can be seen from Table XI, the average weight of the rats in the Utropin® increased on the 1st day, but it decreased on the 2nd day. The weight increased thereafter at a rate similar to that of the control group.

The average weight of the rats in the Comparative Dispersion 2 group continuously increased while that of the rats in the Comparative Emulsion 2 group decreased on the 3rd day. Because the microparticles in Comparative Emulsion 2 have a hydrophilic surface, the drug dissolves, and therefore, it has an weight gain effect equal to that of the aqueous formulation, Utropin®.

In contrast, the average weight of the rats administered with the dispersion of Example 30 and the emulsion of Example 36 increased for 6 days at higher rates than either Comparative Dispersion 2 or Comparative Emulsion 2. The microparticles of the present invention having lecithin coatings are covered with cotton seed oil even after injection, and absorb water only slowly, thereby releasing the drug at a constant rate.

Test Example 13: Animal Test

The dispersion of Example 32 and the emulsion of Examples 41 were each administered to 7 weeks-old female dwarf rats(body weight: approximate 100 g) at a bST dosage of 12.5 mg per rat and then their weight gains were examined. As a control, rats which did not receive bST were used. The average cumulative net weight gains are shown in Table XII.

Table XII

| | | | | | | | | (unit: g) |
|---|---|---|---|---|---|---|---|---|
| Day | 1 | 2 | 3 | 4 | 5 | 6 | 8 | 10 |
| Control | 1.4 | 2.9 | 4.6 | 6.1 | 6.5 | 7.2 | 8.8 | 10.4 |
| Dispersion of Example 32 | 10.3 | 10.8 | 14.9 | 18.1 | 19.4 | 20.6 | 22.2 | 22.7 |
| Emulsion of Example 41 | 9.7 | 11.5 | 14.6 | 17.7 | 19.9 | 20.8 | 22.5 | 22.9 |

As can be seen from Table XII, the average weight of rats administered with either the dispersion of Example 32 or the emulsion of Example 41 increased continuously for 6 days and the rate of daily weight gain was higher than the control group. After 8 days, the weight gain became insignificant, suggesting that the drug release time is about 8 days in both case.

Test Example 14: Cytopathic Effect Inhibition Test

The emulsion of Example 43, Comparative Dispersion 3, interferon-$\alpha$ aqueous formulation were each administered to 5 months-old rabbits (body weight: 2.5 kg) at an interferon-$\alpha$ dosage of 300 $\mu$g.

The blood level of the drug was determined using a cytopathic effect inhibition test, which was conducted by treating cells with interferon-$\alpha$, adding virus thereto and then determining inhibition of the cell pathology. Male calf kidney cell (MDBO CATCC CCF-22) and vesicular stomatitis virus (ATCC VR 158) were used in the test. Titers of interferon-$\alpha$ in the blood were measured for 5 days and the results are shown in Table XIII.

Table XIII

| | 7 hours | day 1 | day 2 | day 3 | day 4 | day 5 |
|---|---|---|---|---|---|---|
| Aqueous Formulation | $1.4 \times 10^3$ | $1.2 \times 10$ | ND* | ND | ND | ND |
| Comparative Dispersion 3 | $2.1 \times 10^3$ | $2.6 \times 10^3$ | $9.1 \times 10^2$ | $3.4 \times 10^2$ | $1.7 \times 10^2$ | $1.5 \times 10$ |
| Emulsion of Example 43 | $1.7 \times 10^3$ | $2.2 \times 10^3$ | $1.1 \times 10^3$ | $4.6 \times 10^2$ | $2.8 \times 10^2$ | $8.7 \times 10$ |
| * not detected | | | | | | |

As can be seen from Table XIII, the titers of interferon-$\alpha$ for the rats administered with the dispersion of Example 43 were high for the entire test period of 5 days, showing higher levels of interferon-$\alpha$ as compared with the Comparative Dispersion 3 group from day 2. Therefore, the dispersion of the present invention has prolonged release characteristics due to the lipophilic surface of the microparticles.

Comparative Test Example 1

hGH was dissolved in 5mM PBS to a concentration of 2.3 mg/m$\ell$ and then sodium hyaluronate having a molecular weight of 2,000,000 was dissolved therein to a concentration of 2 %(w/v) to obtain a hyaluronic acid gel formulation.

An in vitro release test was conducted using the gel formulation by repeating the procedure of Test Example 2. As a result, 100 % of hGH was released into the supernatant within 1 hour. Therefore, this gel formulation has a drug release time which is much shorter than the microparticles of the present invention.

Comparative Test Example 2: Animal Test

The procedure for preparing a hyaluronic acid gel formulation in Comparative Test Example 1 was repeated using 1.5 mg/m$\ell$ hGH to obtain a hyaluronic acid gel formulation having no fluidity.

The gel formulation thus obtained was administered to dwarf rats at a hGH dosage of 150 $\mu$g per rat and then the average weight gain was examined for 6 days. As a comparative formulation, an aqueous solution formulation, Utropin® was administered to rats at the same hGH dosage. As a control, rats which did not receive hGH were used. Results expressed in cumulative weight gains are shown in Table XIV.

Table XIV

| Day / Group | 1 | 2 | 3 | 4 | 5 | 6 |
|---|---|---|---|---|---|---|
| Control | 1.6 | 2.4 | 4.1 | 4.8 | 6.2 | 8.1 |
| Gel Formulation | 3.2 | 3.6 | 3.0 | 6.1 | 6.7 | 7.7 |
| Utropin® | 3.3 | 2.6 | 4.2 | 6.4 | 7.8 | 8.3 |

As can be seen from Table XIV, the average weight gain of the rats administered with the gel formulation is similar to that of the Utropin® group. The rate of weight gain after 2 days was not significantly different among the three groups, suggesting that the drug release from the gel formulation does not lasted more than 1 day.

Comparative Test Example 3

hGH was dissolved in 5mM PBS to a concentration of 2 mg/m$\ell$ and then Tween 80 was added thereto to a concentration of 0.01 wt%. The resulting solution was provided to a spray dryer(Buchi 190) at a flow rate of 3 mℓ/minute to obtain microparticles. In this step, the inflow air temperature was 85 °C. The average particle size of the microparticles thus prepared was 2.5 μm.

Benzyl hyaluronate was dissolved in dimethyl sulfoxide(DMSO) to a concentration of 6 % and then the microparticles were dispersed therein. The resulting dispersion was added to a mineral oil containing Aracel A™(Atlas Chemical Ind.). The mixture was homogenized to obtain a microemulsion. The microemulsion was composed of the mineral oil as a continuous phase and the benzyl hyaluronate/DMSO solution as a dispersed phase.

Ethyl acetate was added to the microemulsion while stirring to extract DMSO from the dispersed phase, giving benzyl hyaluronate microparticles containing hGH. The average particle size of the microparticles was 5.5 μm and the content of hGH was 45 wt%.

An in vitro release test was conducted using the benzyl hyaluronate formulation thus obtained by repeating the procedure of Test Example 2 and the cumulative amounts of hGH released are shown in Table XV.

Table XV

| Hours | 0 | 1 | 3 | 5 | 7 | 24 | 48 | 72 | 144 |
|---|---|---|---|---|---|---|---|---|---|
| Releasing Amount (%) | 0 | 15 | 21 | 23 | 25 | 27 | 28 | 30 | 30 |

As can be seen from Table XV, the benzyl hyaluronate formulation released hGH only slightly after 5 hours and only 30 % of the loaded hGH was released in 144 hours. Thus, most of hGH in the benzyl hyaluronate formulation is bound in the benzyl hyaluronate matrix and not released.

The above benzyl hyaluronate formulation was dispersed in cotton seed oil and the resulting dispersion was administered to dwarf rats at a hGH dosage of 300 μg per rat. The average weight gain was determined and the results expressed in cumulative weight gain are shown in Table XVI.

Table XVI

| Day Formulation | 1 | 2 | 3 | 4 | 5 | 6 | 7 |
|---|---|---|---|---|---|---|---|
| Control | 1.2 | 2.3 | 3.6 | 5.7 | 6.6 | 7.3 | 8.2 |
| Hyaluronic Acid-Benzyl Ester Formulation | 3.6 | 2.7 | 5.4 | 6.3 | 7.1 | 8.4 | 8.0 |

As can be seen from Table XVI, the benzyl hyaluronate formulation shows no significant effect after day 1.

While the invention has been described with respect to the above specific embodiments, it should be recognized that various modifications and changes may be made to the invention by those skilled in the art which also fall within the scope of the invention as defined by the appended claims.

**Claims**

1. A lipophilic microparticle having an average particle size ranging from 0.1 to 200 μm, preferably ranging from 1 to 5 0 μm, comprising a lipophilic substance and an active ingredient selected from the group consisting of a protein or peptide drug and an antigen.

2. The lipophilic microparticle of claim 1, wherein the drug is selected from the group consisting of human growth hormone, bovine growth hormone, porcine growth hormone, growth hormone releasing hormone, growth hormone releasing peptide, granulocyte-colony stimulating factor, granulocyte macrophage-colony stimulating factor, macrophage-colony stimulating factor, erythropoietin, bone morphogenic protein, interferon, insulin, atriopeptin-III, monoclonal antibody, tumor necrosis factor, macrophage activating factor, interleukin, tumor degenerating factor, insulin-like growth factor, epidermal growth factor, tissue plasminogen activator and urokinase.

3. The lipophilic microparticle of claim 1, wherein the antigen is obtained from: one or more pathogens selected from the group consisting of adenovirus type 4&7, hepatitis A virus, hepatitis B virus, hepatitis C virus, influenza A & B virus, Japanese B encephalitis virus, measles virus, epidemic parotitis virus, rubella virus, polio virus, hydrophobia virus, chickenpox virus, yellow fever virus and human immunodeficiency virus; one or more pathogens selected from the group consisting of Bordetellapertussis, Borreliaburgdorferi, enterotoxigenicEscherichiacoli, Haemophil-

usinfluenza type b, <u>Mycobacteriumleprae</u>, <u>Mycobacteriumtuberculosis</u>, <u>Neisseriameningitidis</u> A & C, <u>Neisseriameningitidis</u> B, <u>Pseudomonasaeruginosa</u>, <u>Pseudomonascepacia</u>, <u>Salmonellatyphi</u>, <u>Shigella</u> spp., <u>Streptococcuspneumoniae</u> and <u>Vibriocholerae;</u> one or more pathogens selected from the group consisting of <u>Coccidiodesimmitis</u>, <u>Leishmania</u> sp. and <u>Plasmodium</u> sp.; or one or more pathogens responsible for the disease selected from the group consisting of bovine blackleg, bovine epidemic fever, bovine anthrax, bovine Akabane's disease, bovine foot-and-mouth disease, bovine mammitis, bovine infectious nasotracheal inflammation, bovine viral diarrhea, bovine infectious gastroenteritis, porcine cholera, porcine epidemic diarrhea, porcine atrophic gastritis, porcine disease caused by pavovirus, porcine enteritis caused by rotavirus, chicken Newcastle disease, chicken Marek's disease, chicken encephalomyelitis, rabies, dog distemper, dog enteritis caused by pavovirus and dog infectious hepatitis, the antigen being an attenuated, killed or recombinant antigen; or DNA, RNA, plasmid, CpG DNA or oligonucleotide extracted from the pathogen.

4.  The lipophilic microparticle of claim 1, wherein the lipophilic substance is selected from the group consisting of a lipid, a lipid derivative, a fatty acid, a fatty acid derivative, a wax and a mixture thereof, wherein the lipid preferably is lecithin, phosphatidylcholine, phosphatidylehanolamine or phosphatidylserine, and the lipid derivative preferably is arachidoylphosphatidylcholineor stearoylphosphatidylcholine, and wherein the fatty acid preferably is myristic acid, palmitic acid or stearic acid, and the fatty acid derivative preferably is glyceryl stearate, sorbitanpalmitate, sorbitan stearate, sorbitanmonooleate or polysorbate.

5.  The lipophilic microparticle of claim 1, which further comprises hyaluronic acid or an inorganic salt thereof, wherein the inorganic salt of hyaluronic acid preferably is sodium hyaluronate, potassium hyaluronate, ammonium hyaluronate, calcium hyaluronate, magnesium hyaluronate, zinc hyaluronate or cobalt hyaluronate.

6.  The lipophilic microparticle of any one of claims 1 and 5, which further comprises a water-soluble excipient, wherein the water-soluble excipient preferably is selected from group consisting of a carbohydrate, a protein, an amino acid, a fatty acid, an inorganic salt, a surfactant, poly(ethylene glycol) and a mixture thereof.

7.  A dispersion formulation prepared by dispersing the lipophilic microparticle of any one of claims 1, 5 and 6 in a lipophilic medium.

8.  The dispersion formulation of claim 7, wherein the lipophilic medium is an edible oil, mineral oil, squalene, squalane, cod liver oil, mono-, di- or triglyceride, or a mixture thereof.

9.  The dispersion formulation of claim 8, wherein the edible oil is corn oil, olive oil, soybean oil, safflower oil, cotton seed oil, peanut oil, sesame oil, sunflower oil or a mixture thereof.

10.  The dispersion formulation of claim 7, wherein the lipophilic medium further comprises a dispersing agent or a preservative.

11.  The dispersion formulation of any one of claims 7 and 10, which is used for injection or oral administration.

12.  An oil-in-water emulsion formulation comprising an aqueous injection medium and the dispersion formulation of claim 7.

13.  The oil-in-water emulsion formulation of claim 12, wherein the aqueous injection medium is distilled water or a buffered solution.

14.  The oil-in-water emulsion formulation of claim 12, wherein the active ingredient is an antigen and the aqueous injection medium further comprises a second antigen.

15.  An aerosol formulation comprising the lipophilic microparticle of any one of claims 1 to 6.

# FIG.1

## *FIG.2A*

## *FIG.2B*

## FIG.3A

## FIG.3B

Europäisches
Patentamt

European
Patent Office

Office européen
des brevets

# EUROPEAN SEARCH REPORT

Application Number

EP 11 17 6647

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 41 07 153 A1 (CEVC GREGOR [DE]) 10 September 1992 (1992-09-10) * examples, claims * | 1-15 | INV. A61K9/50 A61K38/00 A61K39/00 |
| X | WO 98/43664 A (LG CHEMICAL LIMITED [KR]; KIM MYUNG JIN [KR]; KIM SUN JIN [KR]; KWON 0) 8 October 1998 (1998-10-08) * examples, claims, esp. claim 10 * | 1-15 | A61K9/10 A61K9/113 A61K9/16 A61K38/21 A61K39/39 A61K38/27 |
| X | EP 0 485 143 A (NACIONAL DE ENGENHARIA E TECNO [PT] INST NACIONAL DE ENGENHARIA E [PT]) 13 May 1992 (1992-05-13) * examples, claims * | 1-15 | |
| X | WO 98/29099 A (BIOVECTOR THERAPEUTICS SA [FR]) 9 July 1998 (1998-07-09) * examples, claims * | 1-15 | |
| A | US 5 059 421 A (LOUGHREY HELEN C [CA] ET AL) 22 October 1991 (1991-10-22) * examples, claims * | 1-15 | |
| A | WO 98/13024 A (HYAL PHARMA CORP [CA]; MARRIOTT CHRISTOPHER [GB]; MARTIN GARY PETER [G) 2 April 1998 (1998-04-02) * examples, claims * | 1-15 | TECHNICAL FIELDS SEARCHED (IPC) A61K |
| Y | WO 92/14449 A1 (NOVA PHARM CORP [US]) 3 September 1992 (1992-09-03) * examples, claims, esp. claim 8 * | 1-15 | |
| Y | WO 95/12385 A1 (ISOTECH MEDICAL INC [US]; CHO YOUNG W [US]) 11 May 1995 (1995-05-11) * claims,esp. claim 30, examples * | 1-15 | |
| A | US 5 858 398 A (CHO YOUNG W [US]) 12 January 1999 (1999-01-12) * the whole document * | 1-15 | |

-/--

The present search report has been drawn up for all claims

| Place of search | Date of completion of the search | Examiner |
|---|---|---|
| Munich | 15 June 2012 | Kronester-Frei, A |

EPO FORM 1503 03.82 (P04C01)

**Europäisches Patentamt**
**European Patent Office**
**Office européen des brevets**

# EUROPEAN SEARCH REPORT

Application Number

EP 11 17 6647

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| L | WO 02/09674 A (ALLIANCE PHARMA [US]; BOT ADRIAN [US]; DELLAMARY LUIS [US]; SMITH DAN) 7 February 2002 (2002-02-07) * examples, claims; the whole document * | 1-15 | |

-----

TECHNICAL FIELDS
SEARCHED     (IPC)

The present search report has been drawn up for all claims

| Place of search | Date of completion of the search | Examiner |
|---|---|---|
| Munich | 15 June 2012 | Kronester-Frei, A |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.....................................................................................................

& : member of the same patent family, corresponding
    document

EPO FORM 1503 03.82 (P04C01)

**ANNEX TO THE EUROPEAN SEARCH REPORT**

**ON EUROPEAN PATENT APPLICATION NO.** EP 11 17 6647

This annex lists the patent family members relating to the patent documents cited in the above-mentioned European search report.
The members are as contained in the European Patent Office EDP file on
The European Patent Office is in no way liable for these particulars which are merely given for the purpose of information.

15-06-2012

| Patent document cited in search report | | Publication date | Patent family member(s) | | Publication date |
|---|---|---|---|---|---|
| DE 4107153 | A1 | 10-09-1992 | NONE | | |
| WO 9843664 | A | 08-10-1998 | AR | 011208 A1 | 02-08-2000 |
| | | | AT | 285245 T | 15-01-2005 |
| | | | AU | 721929 B2 | 20-07-2000 |
| | | | BG | 64642 B1 | 31-10-2005 |
| | | | BG | 102969 A | 30-09-1999 |
| | | | BR | 9804802 A | 17-08-1999 |
| | | | CA | 2256596 A1 | 08-10-1998 |
| | | | CN | 1222857 A | 14-07-1999 |
| | | | DE | 69828252 D1 | 27-01-2005 |
| | | | DE | 69828252 T2 | 10-11-2005 |
| | | | DK | 0918535 T3 | 18-04-2005 |
| | | | EP | 0918535 A1 | 02-06-1999 |
| | | | ES | 2232939 T3 | 01-06-2005 |
| | | | HK | 1020885 A1 | 21-01-2005 |
| | | | HU | 0000737 A2 | 28-09-2000 |
| | | | ID | 20584 A | 21-01-1999 |
| | | | IL | 127220 A | 15-12-2004 |
| | | | JP | 3445283 B2 | 08-09-2003 |
| | | | JP | H11513047 A | 09-11-1999 |
| | | | NZ | 333019 A | 30-08-1999 |
| | | | PL | 330230 A1 | 10-05-1999 |
| | | | PT | 918535 E | 31-03-2005 |
| | | | TR | 9802500 T1 | 21-06-1999 |
| | | | TW | 514532 B | 21-12-2002 |
| | | | US | 2008063727 A1 | 13-03-2008 |
| | | | US | 2011020458 A1 | 27-01-2011 |
| | | | WO | 9843664 A1 | 08-10-1998 |
| | | | ZA | 9802533 A | 30-09-1998 |
| EP 0485143 | A | 13-05-1992 | AT | 161172 T | 15-01-1998 |
| | | | DE | 69128455 D1 | 29-01-1998 |
| | | | DE | 69128455 T2 | 18-06-1998 |
| | | | EP | 0485143 A1 | 13-05-1992 |
| | | | ES | 2110980 T3 | 01-03-1998 |
| WO 9829099 | A | 09-07-1998 | AT | 254911 T | 15-12-2003 |
| | | | AU | 739512 B2 | 11-10-2001 |
| | | | AU | 5675698 A | 31-07-1998 |
| | | | BR | 9713792 A | 08-02-2000 |
| | | | CA | 2275419 A1 | 09-07-1998 |
| | | | DE | 69726450 D1 | 08-01-2004 |
| | | | DE | 69726450 T2 | 16-09-2004 |
| | | | DK | 948322 T3 | 29-03-2004 |
| | | | EP | 0948322 A2 | 13-10-1999 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**                    EP 11 17 6647

This annex lists the patent family members relating to the patent documents cited in the above-mentioned European search report.
The members are as contained in the European Patent Office EDP file on
The European Patent Office is in no way liable for these particulars which are merely given for the purpose of information.

15-06-2012

| Patent document cited in search report | | | Publication date | Patent family member(s) | | | Publication date |
|---|---|---|---|---|---|---|---|
| | | | | ES | 2212145 | T3 | 16-07-2004 |
| | | | | HK | 1025042 | A1 | 25-02-2005 |
| | | | | HU | 0001838 | A2 | 28-10-2000 |
| | | | | JP | 2001507358 | A | 05-06-2001 |
| | | | | US | 6017513 | A | 25-01-2000 |
| | | | | US | 6096291 | A | 01-08-2000 |
| | | | | WO | 9829099 | A2 | 09-07-1998 |
| US | 5059421 | A | 22-10-1991 | NONE | | | |
| WO | 9813024 | A | 02-04-1998 | AU | 740111 | B2 | 01-11-2001 |
| | | | | AU | 4468197 | A | 17-04-1998 |
| | | | | CA | 2266622 | A1 | 02-04-1998 |
| | | | | DE | 69732308 | D1 | 24-02-2005 |
| | | | | DE | 69732308 | T2 | 02-06-2005 |
| | | | | EP | 0963196 | A2 | 15-12-1999 |
| | | | | US | 2001005501 | A1 | 28-06-2001 |
| | | | | WO | 9813024 | A2 | 02-04-1998 |
| WO | 9214449 | A1 | 03-09-1992 | AU | 1442592 | A | 15-09-1992 |
| | | | | US | 5482927 | A | 09-01-1996 |
| | | | | WO | 9214449 | A1 | 03-09-1992 |
| WO | 9512385 | A1 | 11-05-1995 | AT | 198547 | T | 15-01-2001 |
| | | | | CA | 2175494 | A1 | 11-05-1995 |
| | | | | DE | 69426570 | D1 | 15-02-2001 |
| | | | | DE | 69426570 | T2 | 23-08-2001 |
| | | | | DK | 726761 | T3 | 18-06-2001 |
| | | | | EP | 0726761 | A1 | 21-08-1996 |
| | | | | ES | 2155512 | T3 | 16-05-2001 |
| | | | | GR | 3035727 | T3 | 31-07-2001 |
| | | | | HK | 1014150 | A1 | 21-09-2001 |
| | | | | PT | 726761 | E | 31-07-2001 |
| | | | | WO | 9512385 | A1 | 11-05-1995 |
| US | 5858398 | A | 12-01-1999 | NONE | | | |
| WO | 0209674 | A | 07-02-2002 | AU | 8093401 | A | 13-02-2002 |
| | | | | US | 2002106368 | A1 | 08-08-2002 |
| | | | | US | 2005074449 | A1 | 07-04-2005 |
| | | | | US | 2007065369 | A1 | 22-03-2007 |
| | | | | WO | 0209674 | A2 | 07-02-2002 |

EPO FORM P0459

For more details about this annex : see Official Journal of the European Patent Office, No. 12/82

## REFERENCES CITED IN THE DESCRIPTION

**Patent documents cited in the description**

- US 5753234 A
- US 5416017 A
- JP 1287041 A
- JP 2002213 A

**Non-patent literature cited in the description**

- **WEIQI LU et al.** *PDA J. Pharm. Sci. Tech.,* 1995, vol. 49, 13-19
- Biodegradable Polymers as Drug Delivery Systems. Marcel Dekker, 1990
- **HELLER, J.** *Adv. Drug Del. Rev.,* 1993, vol. 10, 163
- **PARK, T. G. et al.** *J. Control. Rel.,* 1995, vol. 33, 211-223
- **LEE, H. K. et al.** *J. Control. Rel.,* 1997, vol. 44, 283-294
- **MEYER, J. et al.** *J. Controlled Rel.,* 1995, vol. 35, 67
- **NIGHTLINGER, N.S. et al.** *Proceed. Intern. Symp. Control Rel. Bioact. Mater.,* 1995
- **ILUM, L. et al.** *J. Controlled Rel.,* 1994, vol. 29, 133
- **LEE, V. H. L.** *Crit . Rev. Ther. Drug Carrier Systems,* 1988, vol. 5, 69-97
- **YOSHIOKA, S. et al.** *J. Pharm. Sci.,* 1982, vol. 71, 593-597
- **SCOTT-MONCRIEFF, J. C. et al.** *J. Pharm. Sci.,* 1994, vol. 83, 1465-1469
- **MURANISHI, S. et al.** *Chem. Pharm. Bull.,* 1977, vol. 25, 1159-1163
- **FIX, J. A. et al.** *Am. J. Physiol.,* 1986, vol. 251, G332-G340
- **SHAO, Z. et al.** *Pharm. Res.,* 1993, vol. 10, 143-250
- **CONSTANTINIDES, P. P. et al.** *Proc. Int. Symp. Control. Release Bioactive Mater.,* 1993, vol. 20, 184-185
- **BJORK, E. et al.** *J. Drug Targeting,* 1995, vol. 2, 501-507
- **GENCO, R. et al.** *Ann. N. Y. Acad. Sci.,* 1983, vol. 409, 650-667